# EUROPÄISCHE PATENTSCHRIFT

(11) **EP 0 865 305 B1**
(45) Veröffentlichungstag und Bekanntmachung des Hinweises auf die Patenterteilung: **02.11.2000**
(21) Anmeldenummer: 96918585.9
(22) Anmeldetag: 18.06.1996
(51) Int. Cl.: A61N 5/06

(54) **BESTRAHLUNGSGERÄT**
RADIATING DEVICE
APPAREIL A RADIATION

(30) Priorität: 20.06.1995 DE 19521998; 03.07.1995 DE 19523616; 06.12.1995 DE 19545389; 21.03.1996 DE 19610996
(43) Veröffentlichungstag der Anmeldung: 23.09.1998
(73) Patentinhaber: Beckmann, Emma, 33611 Bielefeld (DE)
(72) Erfinder: DOPHEIDE, Hans, J., D-33689 Bielefeld (DE)
(74) Vertreter: Beyer, Rudi
(86) Internationale Anmeldenummer: DE9601076
(87) Internationale Veröffentlichungsnummer: WO9700709

(56) Entgegenhaltungen:
- DE-U- 8 900 863
- DE-U- 9 405 934
- DE-U- 29 509 660

## Beschreibung

Die Erfindung betrifft ein Bestrahlungsgerät mit einem Gehäuse, bestehend aus einem Unterteil mit Liegefläche und/oder einer Haube, die einen dazwischenliegenden, mit der Umgebungsluft kommunizierenden freibleibenden Bräunungsraum begrenzen, mit mindestens einer Strahlungsquelle, einer Kühlvorrichtung und einer Stromversorgung, wobei der Gehäuseinnenraum von dem Unterteil und/oder der Haube gegen einen Luftaustausch mit der Umgebungsluft abgeschlossen ist.

Solche Bestrahlungsgeräte sind allgemein bekannt. Sie weisen bei Sonnenbänken unterhalb der Liegefläche und unter der Haube jeweils mehrere Bestrahlungsröhren auf, die üblicherweise aus Quecksilberdampflampen bestehen, die verstärkt UV-Licht abstrahlen. Diese bekannten Bestrahlungsgeräte weisen jedoch einige Nachteile auf, insbesondere, wenn sie in kommerziellen Bräunungsstudios oder Solarien mit eigens dafür konzipierten Bestrahlungsgeräten betrieben werden.

Jedes einzelne dieser Bestrahlungsgeräte erzeugt im Betrieb eine sehr große Wärmemenge, die hier als nicht erwünschte Nebenwirkung angesehen werden muß. Sie wird im wesentlichen durch die Röhren selbst und durch die zu deren Betrieb erforderlichen Vorschaltgeräte, aber auch durch Ventilatormotoren bzw. Kühlaggregate erzeugt, die in diesen bekannten Bestrahlungsgeräten integriert sind. Bei den zur Zeit gängigen Sonnenbänken zum kommerziellen Einsatz liegt diese Wärmemenge je Sonnenbank in einer Größenordnung von 15 - 20 kW. Um eine solche Wärmemenge, wie allgemein üblich, durch Luftkühlung abzuführen, müssen stündlich bis zu 5.600 m³ Luft durch das Bestrahlungsgerät geführt werden, die aus der Raumluft entnommen werden und anschließend wieder in den Raum abgegeben werden. Teilweise werden die Liegeflächen trotzdem so warm, daß zusätzliche, elektrisch betriebene Kühlmaschinen eingebaut werden müssen, deren Abwärme ebenfalls in die Raumluft abgeführt wird. Auf diese Weise kann sich die in einem Sonnenstudio umzuwälzende Luftmenge bei Einsatz von ca. 15 unterschiedlich leistungsstarken Sonnenbänken auf bis zu 50.000 m³ pro Stunde summieren, so daß sich die Raumluft zunehmend erwärmt und Gegenmaßnahmen, wie zum Beispiel eine Klimatisierung der Räume oder die vorübergehende Stillegung einiger Sonnenbänke, getroffen werden müssen. Zusätzlich führt diese starke Luftbewegung in einem Sonnenstudio zu permanenter, das Wohlbefinden der Benutzer oder des Personals negativ beeinflussender Zugluft. Ein weiteres großes Problem stellen bei Zentralabluftsystemen die benötigten Querschnitte der Abluftkanäle dar, die beispielsweise bei einer leistungsfähigen Sonnenbank bereits einen Durchmesser von 0,5m erfordern, so daß bei 15 Sonnenbänken ein mehrere Quadratmeter großer Abluftkanal vorgesehen werden muß. Wenn dies überhaupt baulich möglich ist, so erfordert er sehr viel Platz, der einem Sonnenstudiobetreiber zur Aufstellung von Sonnenbänken verloren geht.

Weiterhin ergibt sich aus den großen umzuwälzenden Luftmassen noch ein anderer gravierender Nachteil, nämlich der, daß diese Luftmassen, auch wenn sie nicht aus der Raumluft, sondern von außerhalb angesaugt werden würden, durch Filter geführt werden müssen, die schnell verschmutzen und demnach oft gesäubert bzw. aufwendig ausgewechselt werden müssen, was aber zu hohen Wartungs- und Personalkosten führt.

Ein weiterer Nachteil besteht darin, daß die zum Transport der Luftmengen benötigten Ventilatoren sehr starke und deswegen störende Geräusche entwikkeln und zudem selbst weitere Abwärme erzeugen und daß insgesamt unnötig viel elektrische Energie dazu verwandt wird, unerwünschte Abwärme abzuführen.

Nachteilig ist weiterhin, daß diese bekannten, überwiegend luftgekühlten Sonnenbänke durch ihre zusätzlichen Einbauten sehr groß und schwer geworden sind, so daß sie sich oftmals nur noch zerlegt transportieren lassen.

Negativ macht sich weiterhin die aufwendige Wartung bzw. das umständliche Auswechseln defekter Röhren bemerkbar, die den Einsatz von geschultem Personal erfordern. Trotz sorgfältiger Behandlung der Röhren können diese in einem Bräunungsstudio zerbrechen und setzen dabei hochgiftiges Quecksilber frei, welches sich leicht in Fußbodenspalten festsetzt und dort langsam verdampft und so ständig eine gesundheitsschädliche Atmosphäre erzeugt. In einem einzigen Sonnenstudio werden zum Teil mehr als 1.000 Röhren betrieben, was bei ca. 2g Quecksilber pro Röhre zu einem enormen Umweltrisiko führt, da eine ordnungsgemäße Entsorgung aus Kostengründen, mangelndem Umweltbewußtsein oder Unkenntnis oftmals unterbleibt.

Als weiterer Nachteil einer Luftkühlung ist anzuführen, daß die im Betrieb entstehende Wärme auch deshalb nicht optimal abgeführt werden kann, weil die Luft selbst bekanntlich ein schlechter Wärmeleiter ist.

Aus der älteren DE 295 09 660 U1 ist ein Bestrahlungsgerät, insbesondere eine Sonnenbank für ein Bräunungsstudio, vorbekannt, mit einem Gehäuse, bestehend aus einem Unterteil mit Liegefläche und/oder einer Haube, mit mindestens jeweils einer Strahlungsquelle, einer Kühlvorrichtung und einer Stromversorgung, wobei der Gehäuseinnenraum gegen einen Luftaustausch mit der Umgebungsluft abgeschlossen ist und eine interne Luftumwälzung aufweist und daß einige oder alle abwärmeerzeugenden und/oder geräuschentwicklenden Bauteile, wie Vorschaltgeräte und Kühlantriebe für die im Gehäuse angeordneten Wärmetauscher, außer den Strahlungsquellen selbst, in einem externen Schaltschrank angeordnet und über Versorgungsleitungen mit dem Bestrahlungsgerät verbunden sind.

Bei diesem Bestrahlungsgerät soll der Schaltschrank außerhalb des Bräunungsstudios, insbesondere im Freien, aufgestellt sein. In dem Schaltschrank können die abwärmeerzeugenden Bauteile mehrerer Bestrahlungsgeräte angeordnet sein. Der externe Schaltschrank kann Lüftungsöffnungen aufweisen, wobei die abwärmeerzeugenden Bauteile durch natürliche Konvektion oder durch eine Kühlvorrichtung kühlbar sind. Beim Einsatz einer Kühlvorrichtung für den Schaltschrank können in dessen Zuluftstrom der luftgekühlte Kondensator eines Kaltwassersatzes angeordnet sein, der für die Versorgung eines Wärmetauschers des Bestrahlungsgerätes mit Kaltwasser dient. Bei einer weiteren Konstruktion dieser Art wird vorgeschlagen, im Bereich der Strahlungsquelle mindestens eine kühlmitteldurchflossene Kühlschlange anzuordnen, die einen Zulauf und einen Ablauf aufweist, über die das Kühlmittel mittels an das Bestrahlungsgerät anschließbarer Versorgungsleitungen einem Wärmetauscher, oder bei Verwendung eines Kältemittels einem Direktverdampfer, zuführbar ist. Weiterhin wird vorgeschlagen, die Kühlung in einem geschlossenen Kühlkreislauf, insbesondere mittels eines luftgekühlten Kaltwassersatzes oder eines Absorbers, vorzunehmen. In den Fällen, in denen das Kühlmittel Wasser ist und die Kühlung in einem offenen Kühlkreislauf erfolgt, wird ein im Freien angeordneter Kühlturm (Verdunstungskühler) vorgeschlagen oder das Kühlmittel soll einem im Sonnenstudio selbst aufgestellten Springbrunnen zugeführt werden.

Aus der DE 89 00 863.4 U1 ist ebenfalls ein Bestrahlungsgerät, insbesondere eine Sonnenbank vorbekannt, die zumindest an der Längsseite des Bestrahlungshimmels Ausströmöffnungen für Luft zur Körperkühlung aufweist. Des weiteren wird im Zusammenhang mit dieser Konstruktion vorgeschlagen, unterhalb der Bestrahlungsliege einen Ventilator vorzusehen.

Die DE 94 05 934.9 U1 beschreibt ebenfalls ein als Liege ausgebildetes Bestrahlungsgerät mit einer Umluftkühlung, bei der die Umluftaggregate mit den Kühleinrichtungen in dem den Unterteil tragenden Sockel angeordnet sind und die Kühlluft zwischen Liegefläche und Bestrahlungsquellen des Unterteiles geführt sind. Das Umkleidungsgehäuse des Unterteils kragt an der Einstiegsseite der Liege balkonartig über die Vorderwand des Sockels hinaus, wobei diese Vorderwand und die darüber vorkragende Wand des Umkleidungsgehäuses Luftaustritts- bzw. Lufteintrittsschlitze aufweisen, und daß den Lufteintrittsschlitzen des Umkleidungsgehäuses Luftleitelemente nachgeordnet sind, die die von den Umluft- und Kühlaggregaten im Sockel erzeugte, aus den Luftaustrittsschlitzen austretende und den Lufteintrittsschlitzen des Umkleidungsgehäuses zugeblasene Luft zwischen der Liegefläche und den Bestrahlungsquellen hindurch einem, an der Rückseite der Liege angeordneten Ansaugaggregat zuleiten, dessen Ausblasseite mit Leitkanälen verbunden ist, die die zugeführte Luft dem Außenraum bzw. den Umluftaggregaten des Sockels zuleiten.

Der Erfindung liegt die Aufgabe zugrunde, ein leichtes, leises und energiesparendes Bestrahlungsgerät zu schaffen, welches auch bei Anordnung mehrerer Bestrahlungsgeräte in einem Raum eines Bräunungsstudios dessen Raumluft nicht ungewollt erwärmt und bei dem die Betriebs-, Wartungs- und Personalkosten, ebenso wie unnötiger Energieverbrauch und sonstige Umweltbelastungen, minimiert werden.

Diese Aufgabe wird durch die in **Patentanspruch 1** wiedergegebenen Merkmale gelöst.

Dadurch, daß bei der Erfindung die betreffende Strahlungsquelle, also die Röhren selbst, von einem flüssigen Kühlmittel umgeben sind, sind voluminöse Zuund Abluftschläuche, die in ein Gehäuse hineingeführt oder aus einem Gehäuse herausgeführt werden müssen, nicht mehr erforderlich. Vielmehr genügen zum Transport der vorhandenen Abwärme relativ dünne Kühlmittelschläuche, oder aber das Volumen des flüssigen Kühlmittels kann so bemessen sein, daß es selbst die sonst unangenehme Wärme der Strahlungsquellen aufnimmt.

Als Kühlmittel kann Wasser verwendet werden, das auch anderen Zwecken zuführbar ist. Zum Beispiel kann das Kühlmittel in einem Kühlturm oder in einem Wärmeaustausch gekühlt und in Umlauf geführt sein. Des weiteren ist es möglich, das erwärmte Kühlmittel auch zu Heizzwecken und zur Raum- und Brauchwassererwärmung heranzuziehen.

**Patentanspruch 2** beschreibt eine erfinderische Ausführungsform, durch welche eine Modulbauweise, also ein Bausatz, möglich ist, mit dem sich die Strahlungsquellen in Form von Röhren mit Schnellkupplungen zu dem gewünschten Strahlungsgerät zusammenbauen lassen. Das Bestrahlungsgerät besteht nur aus einem Aufnahmegerüst für die Module und einem Ständergerüst und den eingesetzten Modulen. Dadurch ist nicht nur ein schneller Austausch defekter Strahlungsquellen und/oder Module möglich, sondern es wird auch die Möglichkeit eröffnet, durch die Modulfertigung Baugrupppen vorzufertigen, um diese am Lager bereitzuhalten, so daß je nach den gewünschten Typen die Bestrahlungsgeräte aus den entsprechenden Modulen zeitsparend zusammengebaut werden können.

**Patentanspruch 3** beschreibt eine weitere erfinderische Ausgestaltung.

Nachfolgend sind einige Ausführungsbeispiele der Erfindung anhand der Zeichnungen näher beschrieben. Es zeigen:
Fig. 1 ein Modul einer Sonnenbank ohne Vorschaltgeräte im Schnitt,
Fig. 2 ein Modul einer Sonnenbank mit Vorschaltgeräten im Schnitt,
Fig. 3 ein Modul mit auf einem Kissen gelagerten Röhren im Schnitt,
Fig. 4 ein Modul mit reiner Flüssigkeitskühlung im Schnitt,
Fig. 5 eine aus mehreren Modulen bestehende Sonnenbank, und
Fig. 6 ein Modul mit doppelwandiger Abdeckung der Liegefläche.

Die dargestellten Ausführungsformen des erfinderischen Bestrahlungsgerätes weisen im wesentlichen einen oberen, die Röhren 1 beinhaltenden Gehäuseteil und einen unteren Gehäuseteil auf. Die Röhren 1 sind auf einem Kissen 2 gelagert, welches so elastisch oder plastisch verformbar ist, daß sich die Kissenhülle an dem Umfang der Röhren 1 anschmiegt. Derjenige Teil der nach oben gerichteten Kissenhülle, der sich nicht mit den Röhren in unmittelbarem Kontakt befindet, ist mit einer reflektierenden Oberfläche 3 ausgestattet, so daß auch seitlich vagabundierende Strahlung nach oben in Richtung der Abdeckung 9, die mit der Liegefläche identisch ist, reflektiert wird.

Das Kissen 2 besteht insgesamt aus gut wärmeleitenden Materialien, die Hülle aus einer Metall- oder Silikonfolie und die Füllung aus einem hochwärmeleitendem Gel oder aus einem flüssigen Kühlmittel 5, welches an einen Kühlkreislauf angeschlossen ist, wozu das Kissen 2 mit einem Zu- und einem Ablauf versehen ist. Anstelle des Kissens 2 kann auch ein Kunststofformteil Verwendung finden, möglicherweise mit einem leitenden Gel als Verbindungsmittel.

Das obere Gehäuseteil ist leicht von dem unteren Gehäuseteil zu trennen, da es nur aufgelegt ist, bzw. mit lösbaren Rasten gehalten und über eine Kontaktleiste mit der nötigen elektrischen Energie für die Röhren 1 versorgt wird. die Vorschaltgeräte 10 können dabei innerhalb oder außerhalb des Gehäuses angeordnet sein.

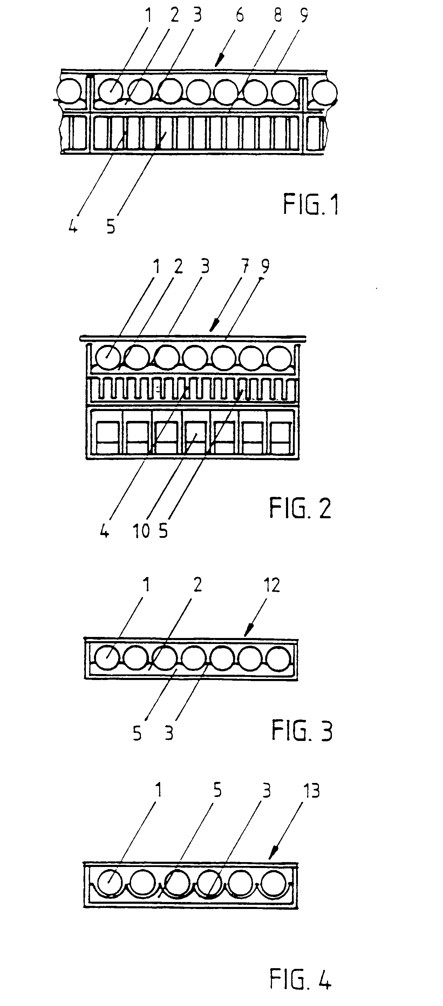

Die Unterseite des oberen Gehäuseteils kann entweder, wie im Modul 6 in Figur 1 dargestellt, mit einer planen Oberfläche 8 ausgestattet sein, die für einen guten Wärmeübergang auf einer ebenfalls planen Oberfläche des unteren, als abgeschlossener Wärmetauscher ausgeführten Gehäuseteils spaltfrei aufliegt, oder sie kann, wie im Modul 7 der Figur 2 gezeigt, mit einer vergrößerten Oberfläche ausgerüstet sein, wie z.B. mit Wärmetauscherflächen 4, die unmittelbar in ein Kühlmittel 5 eintauchen. Im oberen Modul 6 sind diese von einem Kühlmittel 5 umgebenen Wärmetauscherflächen 4 innerhalb des unteren Gehäuseteils angeordnet. In den oberen Modulen 6 befindet sich das Kissen 2 oder das Formbett über den Röhren 1.

Im unteren Gehäuseteil des Moduls 7 sind weiterhin die elektrischen oder elektronischen Vorschaltgeräte 10 der Röhren 1 angeordnet, die ebenfalls von einem Kühlmittel umspült werden, welches identisch mit dem Kühlmittel 5 sein kann, aber auch unterschiedlich und einen eigenen Kühlkreislauf aufweisen kann.

Als gemeinsames Kühlmittel kann beispielsweise ein Gas, insbesondere Luft verwendet werden, die in mindestens einem im Bestrahlungsgerät eingebauten, aber zeichnerisch nicht dargestellten flüssigkeitsbetriebenen Wärmetauscher rückgekühlt wird, und die um die Röhren 1 im Bereich zwischen der Abdeckung 9 und dem Kissen 2 und die Wärmetauscherflächen 4 und evtl. auch um die Vorschaltgeräte 10 zirkuliert.

Denkbar ist insbesondere für eine Ausführungsform nach Figur 1 ein einziges flüssiges Kühlmittel 5 oder bei einer Ausführungsform nach Figur 2 auch die Verwendung eines gasförmigen für die Oberseite der Röhren 1 und die Vorschaltgeräte 10 und eines flüssigen Kühlmittels 5 für die Unterseite des Gelkissens 2, welches um die Wärmetauscherflächen 4 zirkuliert.

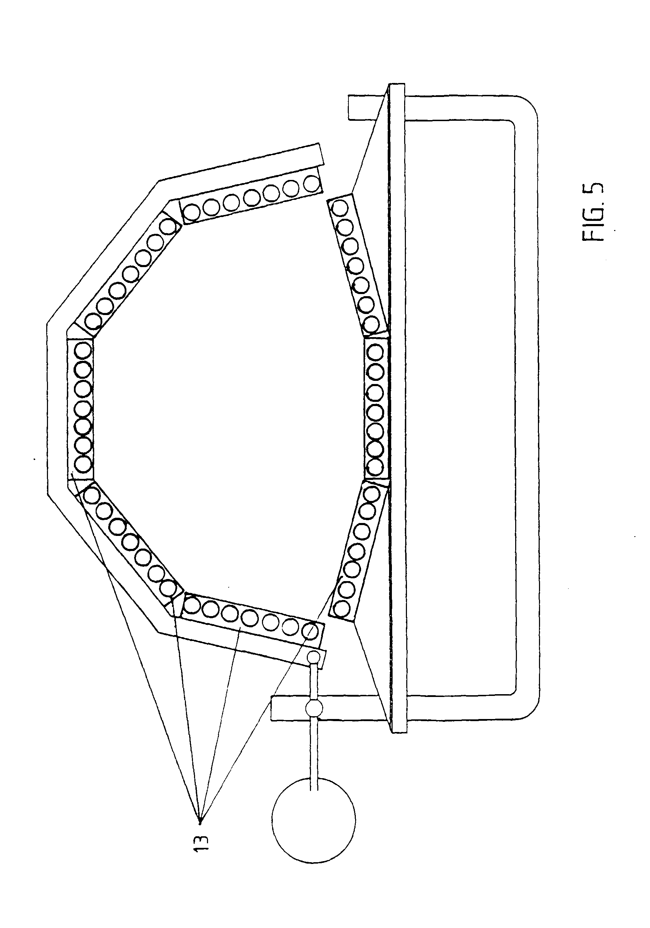

Eine weiter Ausführungsform entsprechend der Figur 4 weist ein rein flüssigkeitsgekühltes Modul 13 auf, bei denen sich die Oberflächen der Röhren 1 und des Gehäuses nicht berühren.

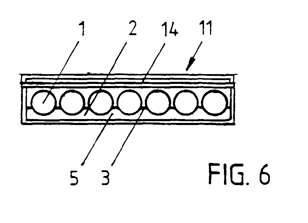

Eine optimierte Ausführungsform eines ebenfalls rein flüssigkeitsgekühlten Moduls 11 des erfinderischen Bestrahlungsgerätes entsprechend der Fig. 6 ist mit einem doppelwandigen Bereich 14 der Liegefläche versehen, so daß diese gezielt und bei Bedarf extra gekühlt oder erwärmt werden kann, insbesondere, daß sie auf einem anderen Temperaturniveau (z.B. 23 °C) wie die Röhren (z.B. 40 °C) gehalten werden kann oder daß zur Kühlung der Liegefläche ein anderes Kühlmittel verwendbar ist als für die Röhren 1.

## Patentansprüche

1. Bestrahlungsgerät mit einem Gehäuse, bestehend aus einem Unterteil mit Liegefläche und/oder einer Haube, die einen dazwischenliegenden, mit der Umgebungsluft kommunizierenden freibleibenden Bräunungsraum begrenzen, mit mindestens einer Strahlungsquelle, einer Kühlvorrichtung und einer Stromversorgung, wobei der Gehäuseinnenraum von dem Unterteil und/oder der Haube gegen einen Luftaustausch mit der Umgebungsluft abgeschlossen ist, **dadurch gekennzeichnet**, daß die Strahlungsquelle auf der der Hauptstrahlungsrichtung entgegengesetzten Seite von einem flüssigen Kühlmittel umgeben ist.

2. Bestrahlungsgerät nach Anspruch 1, **dadurch gekennzeichnet**, daß mehrere Strahlungsquellen in Form von Röhren in einem Bauteil zu einem Modul zusammengefaßt sind, und das Modul und das Bestrahlungsgerät mit Kupplungen für das Kühlmittel und die Energieversorgung versehen sind und daß ein Modul einfach und schnell ein- und ausbaubar ist, wobei das Bestrahlungsgerät nur aus einem Aufnahmegerüst für die Module und einem Ständergerüst und den eingesetzten Modulen besteht.

3. Bestrahlungsgerät nach Anspruch 2, **dadurch gekennzeichnet,** daß mehrere Module zu einer großen Fläche für mehrere Personen zusammengesetzt sind.

## Claims

1. Radiating device with a housing, comprising a bottom part with supporting surface and/or a hood limiting a tanning space in between which remains free and communicates with the ambient air; with at least one radiating source, a cooling device and a power supply, the inner housing space being sealed from the bottom part and/or the hood against an air interchange with the ambient air, **characterized in that** the radiating source is surrounded by a liquid coolant on the side opposite the main direction of radiation.

2. Radiating device as claimed in Claim 1, **characterized in that** several radiating sources in the form of tubes in a component are combined into a module, and in that the module and the radiating device are provided with couplings for the coolant and the supply of energy, and in that a module can be easily and rapidly fitted and removed, the radiating device comprising only a receiving framework for the modules and a pedestal framework and the modules used.

3. Radiating device as claimed in Claim 2, **characterized in that** several modules are combined into one large surface for several persons.

## Revendications

1. Appareil de rayonnement comprenant une carcasse, composée d'une partie inférieure avec une surface de couchage et/ou un capot, délimitant une zone de bronzage dégagée agencée entre eux, communiquant avec l'air environnant, comportant au moins une source de rayonnement, un dispositif de refroidissement et une alimentation en courant, l'espace interne de la carcasse étant isolé de la partie inférieure et/ou du capot contre un échange d'air avec l'air environnant, caractérisé en ce que la source de rayonnement est entourée par un agent réfrigérant liquide sur le côté opposé à la direction de rayonnement principale.

2. Appareil de rayonnement selon la revendication 1, caractérisé en ce que plusieurs sources de rayonnement sont regroupées sous forme de tubes dans un composant, formant un module, le module et l'appareil de rayonnement comportant des raccords pour l'agent réfrigérant et l'alimentation en courant, un module pouvant être monté et démonté de manière simple et rapide, l'appareil de rayonnement se composant uniquement d'un cadre de logement des modules, d'un support et des modules insérés.

3. Appareil de rayonnement selon la revendication 2, caractérisé en ce que plusieurs modules sont assemblés en une grande surface destinée à plusieurs personnes.
